# EUROPEAN PATENT SPECIFICATION

(11) **EP 2 822 553 B1**
(45) Date of publication and mention of the grant of the patent: **18.05.2016**
(21) Application number: 13708351.5
(22) Date of filing: 06.03.2013
(51) Int. Cl.: A61K 31/426, A61K 31/513

(54) **DOSAGE FORM COMPRISING NON-CRYSTALLINE LOPINAVIR AND CRYSTALLINE RITONAVIR**
DARREICHUNGSFORM MIT NICHTKRISTALLINEM LOPINAVIR UND KRISTALLINEM RITONAVIR
FORME GALÉNIQUE COMPRENANT DU LOPINAVIR NON CRISTALLIN ET RITONAVIR CRISTALLIN

(30) Priority: 07.03.2012 EP 12001546
(43) Date of publication of application: 14.01.2015
(73) Proprietor: ratiopharm GmbH, 89079 Ulm (DE)
(72) Inventor: MEERGANS, Dominique, 81379 München (DE); STEFAN, Ralph, 88370 Ebenweiler (DE)
(74) Representative: Ter Meer Steinmeister & Partner
(86) International application number: PCT/EP2013/000658
(87) International publication number: WO 2013/131645

(56) References cited:
- EP-B1- 1 663 183
- US-A1- 2007 027 172
- KAWABATA Y ET AL: "Formulation design for poorly water-soluble drugs based on biopharmaceutics classification system: Basic approaches and practical applications", INTERNATIONAL JOURNAL OF PHARMACEUTICS 20111125 ELSEVIER NLD LNKD- DOI:10.1016/J.IJPHARM.2011.08.032, vol. 420, no. 1, 25 November 2011 (2011-11-25), pages 1-10, XP002675398, ISSN: 0378-5173
- SANDRIEN JANSSENS ET AL: "Review: physical chemistry of solid dispersions", JOURNAL OF PHARMACY AND PHARMACOLOGY, vol. 61, no. 12, 1 December 2009 (2009-12-01), pages 1571-1586, XP055023760, ISSN: 0022-3573, DOI: 10.1211/jpp/61.12.0001
- KANZER J ET AL: "In situ formation of nanoparticles upon dispersion of melt extrudate formulations in aqueous medium assessed by asymmetrical flow field-flow fractionation", JOURNAL OF PHARMACEUTICAL AND BIOMEDICAL ANALYSIS, NEW YORK, NY, US, vol. 53, no. 3, 2 November 2010 (2010-11-02), pages 359-365, XP027147796, ISSN: 0731-7085 [retrieved on 2010-04-24]

## Description

### Background of the Invention

The present invention relates to an oral dosage form comprising non-crystalline lopinavir and crystalline ritonavir. The invention further relates to methods of preparing said oral dosage forms containing the above pharmaceutical active agents.

"Lopinavir" is reported to be the INN name of (2*S)-N-[(*2*S,*4*S,*5*S)-*5-[2-(2,6-dimethylphenoxy)acetamido]-4-hydroxy-1,6-diphenylhexan-2-yl]-3-methyl-2-(2-oxo-1,3-diazinan-1-yl)butanamide and is characterized by the following chemical formula (I):

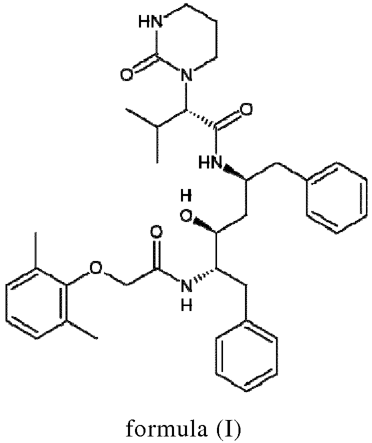

Lopinavir is reported to be an antiretroviral active substance, being a member of the protease inhibitors (PI), which are used to treat or prevent infection caused by viruses. Proteases are enzymes used by viruses to cleave proteins for the final assembly of new virions. In the case of lopinavir, especially the prevention of viral replication by inhibiting the activity of proteases, such as HIV-1 protease, are reported.

"Ritonavir" is reported to be the INN name of 1,3-thiazol-5-ylmethyl N-[(2*S*,3*S*,5*S*)-3-hydroxy-5-[(2*S*)-3-methyl-2-{[methyl({[2-(propan-2-yl)-1,3-thiazol-4-yl]methyl})carbamoyl]amino}butanamido]-1,6-diphenylhexan-2-yl]carbamate and is characterized by the following chemical formula (II):

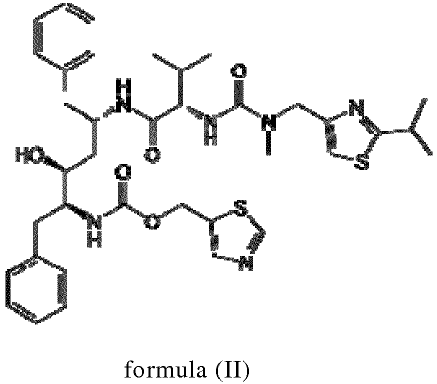

Ritonavir is also reported to be a member of the class of protease inhibitors and is used in the treatment of HIV infection and AIDS. However, ritonavir is frequently described to be used in a combination with other antiretroviral drugs, due to its feature to inhibit the same host enzyme that metabolizes other protease inhibitors. Due to this inhibition of the above host enzyme, the plasma concentrations of the further protease inhibiting drugs tend to be higher, so that their dose and frequency in administration can be lowered.

EP 1 663 183 B1 describes a solid pharmaceutical composition comprising ritonavir. The pharmaceutical composition can optionally comprise further protease inhibitors, such as lopinavir, indinavir and saquinavir. However, it turned out that the compositions described in the art, show a dissolution and plasma profile, which can be improved, especially during the first 30 minutes after administration. Also content uniformity of those compositions is still improvable.

US 2007/027172 A1 (DICKMANN DANIEL A. [US] ET AL) relates to novel crystalline forms of (2S,3S,5S)-2-(2,6-dimethylphenoxyacetyl)amino-3-hydroxy-5-(2-(1-tetrahydropyrimid-2-onyl)-3-methylbutanoyl)amino-1,6-diphenylhexane (also known as lopinavir) and methods for their preparation. The crystalline forms can be used to purify or isolate lopinavir or for the preparation of pharmaceutical compositions for the administration of lopinavir.

KAWABATA Y ET AL: "Formulation design for poorly water-soluble drugs based on biopharmaceutics classification system: Basic approaches and practical applications", INTERNATIONAL JOURNAL OF PHARMACEUTICS 20111125 ELSEVIER NLD LNKD-DOI: 10.1016/J.IJPHARM.2011.08.032, vol. 420, no. 1, 25 November 2011 (2011-11-25), pages 1-10, XP002675398, ISSN: 0378-5173 describes the basic approaches for enhancing the solubility of poorly water-soluble drugs, such as crystal modification, micronization, amorphization, self-emulsification, cyclodextrin complexes and pH-modification.

SANDRIEN JANSSENS ET AL: "Review: physical chemistry of solid dispersions", JOURNAL OF PHARMACY AND PHARMACOLOGY, vol. 61, no. 12, 1 December 2009 (2009-12-01), pages 1571-1586, XP55023760, ISSN:0022-3573, DOI: 10.1211/jpp/61.12.0001 relates to the physical chemistry of solid dispersions, in particular to solid dispersion of poorly water-soluble drugs to enhance the their bioavailability.

KANZER J. ET AL.: "In situ formation of nanoparticles upon dispersion of melt extrudate formulations in aqueous medium assessed by asymmetrical flow field-flow fractionation", JOURNAL OF PHARMACEUTICAL AND BIOMEDICAL ANALYSIS, NEY YORK, NY, US, vol. 53, no. 3, 2 November 2010 (2010-11-02), pages 359-365, XP027147796, ISSN: 0731-7085 relates to the in situ formation of nanoparticles upon dispersion of melt extrudate formulations in aqueous medium. It is described that during hot-melt extrusion processes solid dispersion may be formed and that several of these formulation are reported to have enhanced bioavailability. A search on the mechanism behind is carried out.

Further, it turned out that the known compositions have to be processed within a very small and specific range of process parameters, i.e. the manufacturing process and thus the quality of the resulting products is device-dependent.

Additionally, the storage stability of the prior art compositions is often not satisfactory, especially when stored under conditions of climate zones III and IV. These climate zones are characterized by a temperature of 30°C and a relative humidity of 35% (climate zone III) and of 70% (climate zone IV).

Hence, it was an object of the present invention to overcome the drawbacks of the prior art compositions. Consequently, an oral dosage form comprising a combination of lopinavir and ritonavir and having superior in-vitro and in-vivo properties should be provided, preferably in combination with excellent content uniformity. Any food effect should be minimized. In particular, an oral dosage form should be provided with improved in-vitro properties, such as excellent dissolution within the first 45 minutes. Further, in the dissolution profile, any lag time should be prevented. The lag time should preferably be prevented even in case the oral dosage form is coated with a commercially obtainable HPMC-coating. The dosage form should comprise only minor amounts of decomposition products. Those advantages should be achievable even under the harsh storage conditions of climate zones III and IV. Further, the dosage form should be producable by a predominantly device-independent manufacturing process.

### Summary of the Invention

According to the present invention, the above objects are solved by an oral dosage form comprising non-crystalline lopinavir and crystalline ritonavir and by a process for producing said dosage form.

Thus, a subject of the present invention is an oral dosage form comprising (a) non-crystalline lopinavir and (b) crystalline ritonavir.

It was found that the oral dosage form of the present invention leads to superior in-vitro and in-vivo properties, for example a superior dissolution profile (in particular within the first 45 minutes) and to superior plasma levels. Further, an improved content uniformity of the drug can be achieved, which can ensure that the appropriate dose can be applied to the patient. The advantages were achievable even after a long storage period under harsh conditions without significant amounts of decomposition.

Another subject of the invention relates to a method for preparing the oral dosage form of the present invention comprising
(i) providing lopinavir, optionally vehicle and/or pharmaceutical excipient(s),
(ii) optionally processing the mixture of step (i) to assure that lopinavir is obtained in a non-crystalline form,
(iii) optionally granulating the pharmaceutical composition from step (ii),
(iv) mixing the lopinavir of step (i), the mixture of step (ii) or the granules of step iii) with crystalline ritonavir and optionally pharmaceutical excipients,
(v) processing the mixture of step (iv) into an oral dosage form.

It was found that the method of the present invention can be conducted without being bound to very specific process parameters or specific devices. For example, in case lopinavir has to be melted for assuring that it is obtained in a non-crystalline form, this process can be conducted in a melt extruder as well as in a conventional heatable vessel. Contrary, the prior art process for producing amorphous lopinavir/ritonavir necessarily requires quite specific devices and process parameters.

### Detailed Description of the Invention

In the context of this invention, the term "lopinavir" usually refers to *(2S)-N-*[(2*S*,4*S*,5*S*,)-5-[2-(2,6-dimethylphenoxy)acetamido]-4-hydroxy-1,6-diphenylhexan-2-yl]-3-methyl-2-(2-oxo-1,3-diazinan-1-yl)butanamide in accordance with formula (I) above. In addition, the term "lopinavir" as used in the present application can refer to free lopinavir as well as to its pharmaceutically acceptable salts, hydrates, solvates, polymorphs and mixtures thereof.

In a preferred embodiment of the present invention lopinavir is used in the form of the free lopinavir, i.e. as shown in formula (I).

Generally, the term "non-crystalline" refers to any solid state being non-crystalline. Preferably, non-crystalline lopinavir means amorphous lopinavir, lopinavir in form of a solid dispersion or solid solution. Amorphous lopinavir as compound (a) is particularly preferred.

The term "amorphous" can be used in the context of this invention to designate the state of solid substances, in which the components (atoms, ions or molecules, i.e. in the case of amorphous lopinavir the lopinavir molecules) do not exhibit any periodic arrangement over a great range (= long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (= short-range order). Consequently, amorphous substances preferably possess a short-range order but no long-range order.

In contrast to anisotropic crystals, solid non-crystalline substances can be isotropic. Normally, they do not have a defined melting point, but instead pass over into the liquid state after slowly softening. They can be distinguished from crystalline substances experimentally by means of X-ray diffraction, which does not reveal clearly defined interferences for them, but rather, in most cases, only a few diffuse interferences with small diffraction angles.

The non-crystalline lopinavir (a) in the oral dosage form of the invention may consist of pure non-crystalline lopinavir (a). Alternatively, it may also contain small amounts of crystalline lopinavir components, provided that no defined melting point of crystalline lopinavir can be detected in a DSC. A mixture is preferred, containing 60 to 99.999% by weight of non-crystalline lopinavir (a) and 0.001 to 40% by weight of crystalline lopinavir, more preferably the mixtures contains 90 to 99.99% by weight of non-crystalline lopinavir (a) and 0.01 to 10% of crystalline lopinavir, particularly preferably 95 to 99.9% by weight of non-crystalline lopinavir (a) and 0.1 to 5% of crystalline lopinavir.

In the context of this invention, the term "ritonavir" usually refers to (1,3-thiazol-5-ylmethyl *N*-[(2*S*,3*S*,5*S*)-3-hydroxy-5-[(2*S*)-3-methyl-2-{[methyl({[2-(propan-2-yl)-1,3-thiazol-4-yl]methyl})carbamoyl] amino }butanamido]-1,6-diphenylhexan-2-yl]carbamate in accordance with formula (II) above. In addition, the term "ritonavir" as used in the present application can refer to ritonavir in the form of the free base as well as to its pharmaceutically acceptable hydrates, salts, solvates, polymorphs and mixtures thereof.

In a preferred embodiment of the present invention the crystalline ritonavir (b) in the oral dosage form can preferably be polymorphic Form I of ritonavir. A method for producing Form I is disclosed in EP 1 097 148 B1. In the present application, Form I is characterized by the following two-theta angle positions of the characteristic peaks in x-ray powder diffraction (XRPD):
3.3°±0.1°, 8.3°±0.1°, 18.1°±0.1°, 21.5°±0.1°

Further characteristic peaks can be found:
6.8° ± 0.1°, 19.5° ± 0.1°, 23.5° ± 0.1°, 24.4° ± 0.1°

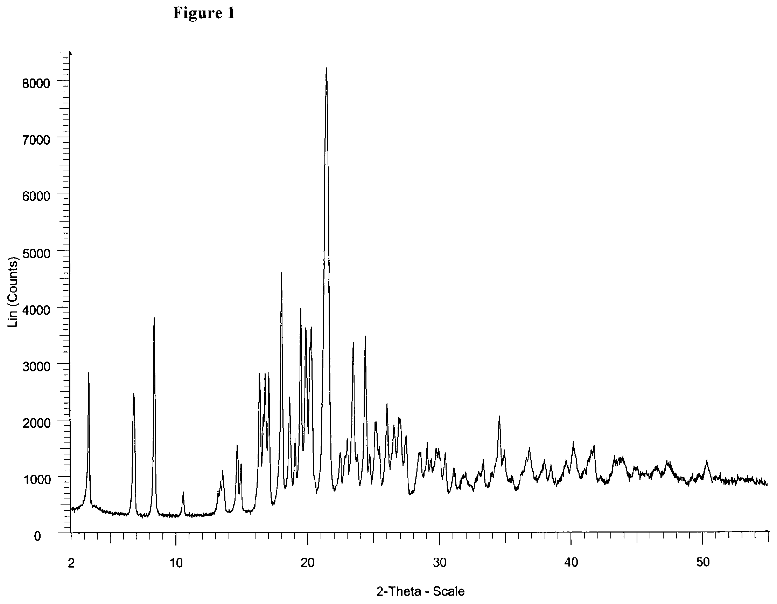

An XRPD of ritonavir Form I is shown in Figure 1.

The x-ray diffraction diagrams of the powders are obtained in reflexion configuration (Bragg-Brentano-Geometry). Polymethylmethacrylate (PMMA) carriers are used as sample carrier, with a sample chamber of 20.0 mm in diameter and 1 mm depth. Measurements are performed by means of an x-ray source with copper anode at a generator voltage of 40 KV and 40 mA electric current in a measure circuit of 435.0 mm. The detection is carried out with a fast, highly sensitive and position-sensitive detector (Vantec-1 of Fa. Bruker AXS, Karlsruhe).

It has been unexpectedly found, that the above-mentioned problems can be advantageously solved when ritonavir Form I is used, especially since Form II was reported to be more stable.

The term "crystalline" can be used in the context of this invention to designate the state of solid substances, in which the components (atoms, ions or molecules, i.e. in the case of crystalline ritonavir the ritonavir molecules) are arranged in an orderly repeating pattern, extending in all three spatial dimensions and thus exhibits a periodic arrangement over a great range (= long-range order).

The crystalline ritonavir (b) in the oral dosage form of the invention may consist of purely crystalline ritonavir (b). Alternatively, it may also contain small amounts of non-crystalline ritonavir components, provided that a defined melting point of crystalline lopinavir can be detected in a DSC. A mixture containing 85 to 99.999% by weight crystalline ritonavir (b) and 0.001 to 15% by weight non-crystalline ritonavir is preferred, more preferably 90 to 99.99% by weight crystalline ritonavir (b) and 0.01 to 10% non-crystalline ritonavir, particularly preferably 95 to 99.9% by weight crystalline ritonavir (b) and 0.1 to 5% non-crystalline ritonavir.

The crystalline ritonavir comprised in the oral dosage form of the present invention can have an average particle size (D50) of 0.5 to 150 µm, preferably 0.7 to 75 µm, more preferably 1.0 to 20 µm, particularly preferably 1.2 to 10 µm.

Further, the crystalline ritonavir comprised in the oral dosage form can have a D10-value of the particle size distribution of 0.1 to 15 µm, preferably 0.2 to 7 µm, more preferably 0.3 to 3 µm, particularly preferably 0.4 to 1 µm.

Further, the crystalline ritonavir comprised in the oral dosage form can have a D90-value of the particle size distribution of 2 to 250 µm, preferably 5 to 100 µm, more preferably 7 to 40 µm, particularly 10 to 25 µm.

The term "average particle size" usually refers to the D50-value of the particle size distribution. The particle distribution can be determined by means of laser diffractometry. In particular, a Malvern Instruments Mastersizer 2000 can be used to determine the size (preferably wet measurement with ultrasound 60 sec., 2,000 rpm, preferably dispersed in water, obscuration 4-6%, the evaluation being performed according to Mie Model).

The average particle size (D50), which is also denoted D50-value of the integral volume distribution, is defined in the context of this invention as the particle diameter, at which 50 percent by volume of the particles have a smaller diameter than the diameter which corresponds to the D50-value. Likewise, 50 percent by volume of the particles have a larger diameter than the D50-value. Analogously, the D90-value of the integral volume distribution is defined as the particle diameter, at which 90 percent by volume of the particles have a smaller diameter than the diameter, which corresponds to the D90-value. Correspondingly, the D10-value of the integral volume distribution is defined as the particle diameter, at which 10 percent by volume of the particles have a smaller diameter than the diameter, which corresponds to the D10-value.

In a particularly preferred embodiment the oral dosage form of the present invention comprises the combination of lopinavir and ritonavir as sole pharmaceutical active agents. In an alternative embodiment the oral dosage form of the invention can comprise lopinavir and ritonavir in combination with further pharmaceutical active agent(s). In case that the oral dosage form of the invention comprises lopinavir and ritonavir in combination with further pharmaceutical active agents, the further pharmaceutical active agent(s) is preferably selected from zidovudine, lamivudin, tenofovir and/or abacavir.

Preferably, the oral dosage form of the present invention comprises 20 mg to 500 mg lopinavir, more preferably 30 mg to 400 mg lopinavir, still more preferably 40 mg to 300 mg lopinavir, particularly preferably 50 mg to 250 mg lopinavir. The amounts generally refer to "free" lopinavir (i.e. when lopinavir is present in form of a salt or a solvate, the corresponding amount has to be added accordingly).

Preferably, the oral dosage form of the present invention comprises 5 mg to 150 mg ritonavir, more preferably 10 mg to 125 mg ritonavir, still more preferably 15 mg to 100 mg ritonavir, particularly preferably 20 mg to 75 mg ritonavir. The amounts generally refer to "free" ritonavir (i.e. when ritonavir is present in form of a salt or a solvate, the corresponding amount has to be added accordingly).

In a preferred embodiment of the invention, the non-crystalline lopinavir (a) is present in a mixture with a vehicle (c). The term "vehicle (c)" may refer to a single vehicle (c) or a mixture of more than one vehicle (c). The non-crystalline lopinavir (a) in the mixture with the vehicle (c) can be regarded as lopinavir (a) in a stabilized form. This mixture can be regarded as a pharmaceutical intermediate. The intermediate preferably is further processed to give the final oral dosage form.

In a preferred embodiment the oral dosage form of the invention can comprise lopinavir (a) and vehicle (c), wherein the weight ratio of non-crystalline lopinavir (a) to vehicle (c) can be from 1:10 to 10:1, preferably from 1:7 to 7:1, more preferably from 1:5 to 5:1 and particularly from 1:3 to 2:1.

In a preferred embodiment the vehicle (c) can be present in an amount of 5 to 75 wt.%, preferably 10 to 70 wt.%, more preferably 15 to 65 wt.%, based on the total weight of the oral dosage form.

Generally, the vehicle (c) can be a substance, which is capable of inhibiting the recrystallisation of non-crystalline to crystalline lopinavir.

In a preferred embodiment, the vehicle (c) is a substance, which inhibits the recrystallisation of non-crystalline to crystalline lopinavir. Preferably, the recrystallisation is inhibited such that not more than 20%, preferably not more than 10% of the non-crystalline lopinavir, present at the time of manufacture (TO), is transformed into crystalline lopinavir upon storage for three months at 45°C / 75% relative humidity (RH).

Generally, the vehicle (c) can be a non-brittle or brittle substance.

Pharmaceutical excipients, such as vehicles, can generally be classified with regard to the change in the shape of the particles under compression pressure (compaction): plastic excipients are characterised by plastic deformation, whereas when compressive force is exerted on brittle substances, the particles tend to break into smaller particles. Brittle behaviour on the part of the substrate can be quantified by the increase in the surface area in a moulding. In the art, it is customary to classify the brittleness in terms of the "yield pressure". According to a simple classification, the values for the "yield pressure" here are low for plastic substances but high in the case of friable substances (Duberg, M., Nyström, C., 1982, "Studies on direct compression of tablets VI. Evaluation of methods for the estimation of particle fragmentation during compaction.", Acta Pharm. Suec. 19, 421-436; Humbert-Droz P., Mordier D., Doelker E., "Méthode rapide de determination du comportement a la compression pour des études de préformulation.", Pharm. Acta Helv., 57, 136-143 (1982)). The "yield pressure" describes the pressure that has to be reached for the excipient (i.e. preferably the vehicle) to begin to flow plastically.

The "yield pressure" is preferably calculated by using the reciprocal of the gradient of the Heckel plot, as described in York, P., Drug Dev. Ind. Pharm. 18, 677 (1992). The measurement in this case is preferably made at 25°C and at a deformation rate of 0.1 mm/s.

In the context of the present invention, an excipient (especially a vehicle) is deemed a non-brittle excipient when it has a "yield pressure" of not more than 120 MPa, preferably not more than 100 MPa, particularly preferably 5 to 80 MPa. An excipient is usually described as a brittle excipient when it has a "yield pressure" of more than 80 MPa, preferably more than 100 MPa, particularly preferably more than 120 MPa, especially more than 150 MPa. Brittle excipients may exhibit a "yield pressure" of up to 300 MPa or up to 400 MPa or even up to 500 MPa.

Examples of non-brittle excipients (vehicles) are mannitol, povidone, copovidone or starch.

Examples of brittle excipients (vehicles) are microcrystalline cellulose, calcium hydrogen phosphate, silicates or aluminosilicates, preferably magnesium aluminosilicates.

In a particularly preferred embodiment, brittle substances are used as a vehicle (c) in the oral dosage form of the present invention. It is further preferred that brittle and non-water soluble substances are used as vehicle (c).

In a preferred embodiment of the invention the vehicle (c) comprises an inorganic substance, preferably a non-brittle inorganic substance and/or a non-water-soluble inorganic substance. A non-water-soluble substance generally is a pharmaceutical excipient, as specified in the European Pharmacopoeia, with a water solubility of less than 33 mg/ml, measured at 25°C. Preferably, the non-water-soluble substance has a solubility of 10 mg/ml or less, more preferably 5 mg/ml or less, especially 0.01 to 2 mg/ml (determined according to Column Elution method pursuant to EU Directive RL67-548-EWG, Appendix V Chapt. A6).

In a further preferred embodiment the inorganic substance (c) can be a phosphate or a silicate, preferably a silicate, more preferably an aluminosilicate. Especially preferred is dicalcium phosphate (e.g. Dicafos AN), or magnesium aluminosilicate, for example Al₂O3· MgO· 1.7SiO₂·xH₂O.

In a preferred embodiment the vehicle (c), in particular the inorganic substance (c), has a specific surface area of 50 to 450 m²/g, more preferably 75 to 400 m²/g, in particular 100 to 300 m²/g. The specific surface area preferably is determined by gas adsorption according to Ph. Eur., 6^{th} edition, Chapter 2.9.26. For this purpose, an ASAP^{®} 2020 (Micromeritics) and an 'outgasing' temperature of 40°C is used. It has surprisingly been found that the above-mentioned specific surface area might be beneficial for achieving the above-mentioned objects (e.g. superior in-vitro and in-vivo properties), in particular, since substances with a high specific surface area are reported to reduce the bioavailability of lopinavir.

In a preferred embodiment, silica, such as Aerosil^{®} 200, does not work as vehicle (c).

In an alternative embodiment of the invention the vehicle (c) can preferably be an organic polymer. In addition, the vehicle (c) can also include substances which behave like polymers. Examples of these substances are fats and waxes. Furthermore, the vehicle (c) can also include solid, non-polymeric compounds, which preferably can contain polar side groups. Examples of these compounds are sugar alcohols or disaccharides.

In a preferred embodiment the vehicle (c) can be a polymer. The polymer to be used for the preparation of the pharmaceutical composition preferably may have a glass transition temperature (Tg) of more than 45°C, more preferably 50°C to 150°C, in particular from 55°C to 120°C. A respective Tg can be important for achieving the desired properties of the resulting dosage form.

In the present invention, the term "glass transition temperature" (Tg) describes the temperature at which amorphous or partially crystalline polymers change from the solid state to the liquid state. In the process, a distinct change in physical parameters, e.g. hardness and elasticity, occurs. Beneath the Tg, a polymer is usually glassy and hard, whereas above the Tg, it changes into a rubber-like to viscous state. The glass transition temperature is determined in the context of this invention by means of dynamic differential scanning calorimetry (DSC).

For this purpose, a Mettler Toledo^{®} DSC 1 apparatus can be used. The work is performed at a heating rate of 1-20°C/min, preferably 10°C/min, and at a cooling rate of 5°C to 50°C/min, preferably 50°C/min.

In general, the organic polymer to be used as vehicle (c) preferably can have a weight-average molecular weight of 1,000 to 500,000 g/mol, more preferably from 1,500 to 100,000 g/mol and particularly from 2,000 to 50,000 g/mol. The weight-average molecular weight is preferably determined by means of gel permeation chromatography.

If the organic polymer used as vehicle (c) is dissolved in water in an amount of 2% by weight, the resulting solution preferably can have a viscosity of 1 to 50 mPa·s, more preferably 1.5 to 20 mPa·s, and even more preferably from 2 to 12 mPa·s or (especially in the case of HPMC) from 12 to 18 mPa·s, measured at 25°C, and determined in accordance with Ph. Eur. 6.0, Chapter 2.2.10.

In the present invention, hydrophilic polymers can preferably be used as vehicle (c). The term "hydrophilic polymers" generally refers to polymers, which possess hydrophilic groups. Examples of suitable hydrophilic groups can be hydroxy, sulfonate, carboxylate and quaternary ammonium groups.

The vehicle (c) may, for example, comprise the following polymers: polysaccharides, such as hydroxypropyl methyl cellulose (HPMC), carboxymethyl cellulose (CMC, especially sodium and calcium salts), ethyl cellulose, methyl cellulose, hydroxyethyl cellulose, ethyl hydroxyethyl cellulose, hydroxypropyl cellulose (HPC), hydroxypropyl methyl cellulose acetate succinate (HPMCAS), hydroxypropyl methyl cellulose succinate (HPMCS), hydroxypropyl cellulose acetate succinate (HPCAS), hydroxyethyl methyl cellulose succinate (HEMCS), hydroxyethyl cellulose acetate succinate (HECAS), hydroxypropyl methyl cellulose phthalate (HPMCP), hydroxyethyl methyl cellulose acetate succinate (HEMCAS), carboxymethyl cellulose (CMC), polyvinylpyrrolidone, polyvinyl alcohol, polymers of acrylic acid and their salts, vinyl pyrrolidone/vinyl acetate copolymers (such as Kollidon^{®} VA 64, BASF), gelatine polyalkylene glycols, such as polypropylene glycol or preferably polyethylene glycol, gelatine and mixtures thereof.

The vehicle (c) preferably used can be polyvinylpyrrolidone, preferably with a weight-average molecular weight of 10,000 to 60,000 g/mol, especially 12,000 to 40,000 g/mol, vinylpyrrolidone and vinyl acetate copolymer, especially with a weight-average molecular weight of 45,000 to 75,000 g/mol and/or polymers of acrylic acid and their salts, especially with a weight-average molecular weight of 50,000 to 250,000 g/mol. In addition, HPMC can preferably be used, especially with a weight-average molecular weight of 20,000 to 90,000 g/mol and/or preferably a proportion of methyl groups of 10 to 35% and a proportion of hydroxy groups of 1 to 35%. Likewise, HPC can be preferably used, especially with a weight-average molecular weight of 50,000 to 100,000 g/mol. Also, polyethylene glycol with a weight-average molecular weight of 2,000 to 40,000 g/mol, especially from 3,500 to 25,000 g/mol, can preferably be used. Likewise, a polyethylene/polypropylene block copolymer can preferably be used, wherein the polyethylene content can preferably be 70 to 90% by weight. The polyethylene/polypropylene block copolymer preferably has a weight-average molecular weight of 1,000 to 30,000 g/mol, more preferably from 3,000 to 15,000 g/mol. More preferably, microcrystalline cellulose as well as silicified microcrystalline cellulose can be used, especially when it possesses a weight average molecular weight of 100,000 to 750,000 g/mol, in particular 125,000 to 650,000 g/mol. The weight-average molecular weight can usually be determined by means of gel permeation chromatography.

In a preferred embodiment, the vehicle (c) used can be a copolymer of vinylpyrrolidone and vinyl acetate, especially with a weight-average molecular weight of 45,000 to 75,000 g/mol. The copolymer can be characterised by the following structural formula (III):

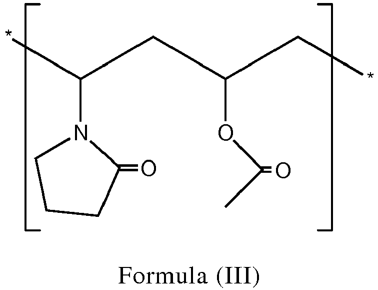

Likewise, it can preferably be possible to use sugar alcohols such as mannitol, sorbitol, xylitol as vehicles (c).

In a preferred embodiment the oral dosage form can further comprise one or more pharmaceutical excipient(s) (d).

Examples of pharmaceutical excipients are glidants, fillers, binders, disintegrants, surfactants and lubricants.

Glidants can be used to improve the flowability. For example, talc can be used as glidant. More preferably, colloidal silica (for example Aerosil^{®}) is used. Preferably, the glidant can be present in an amount of up to 3 wt.%, in particular, 0.1 to 2 wt.%, based on the oral dosage form. Preferably, the silica has a specific surface area of 50 to 400 m²/g, measured by gas adsorption according to Ph. Eur., 6.0, Chapter 2.9.26.

Fillers can be used to increase the bulk volume and weight of a low-dose drug to a limit at which a pharmaceutical dosage can be formed. Fillers may fulfil several requirements, such as being chemically inert, non-hygroscopic, biocompatible, easily processable and may possess good biopharmaceutical properties. Examples of fillers are lactose, sucrose, glucose, mannitol, calcium carbonate, cellulose and others.

The fillers can be present in the oral dosage form of the present invention in an amount of 0 to 50 wt.%, preferably 1 to 35 wt.%, more preferably 5 to 30 wt.% and still more preferably 10 to 25 wt.% of the total weight of the oral dosage form.

Binders usually are regarded as substances for ensuring that the oral dosage form (in particular the tablet) can be formed with the required mechanical strength. In the present invention preferably organic polymers, which are described above as vehicle (c), also act as binders.

Disintegrants usually are compounds, which can enhance the ability of the intermediate to break into smaller fragments when in contact with a liquid, preferably water. Preferred disintegrants are sodium carboxymethyl starch, crosslinked polyvinylpyrrolidone (Crospovidone), sodium carboxymethyl glycolate (for example Explotab^{®}), swelling polysaccharide, for example soy polysaccharide, carrageenan, agar, pectin, starch and derivates thereof, protein, for example formaldehyde-casein, sodium bicarbonate or mixtures thereof. Crospovidone is particularly preferred.

The disintegrant can be present in the oral dosage form of the present invention in an amount of 0 to 20 wt.%, preferably 1 to 17 wt.%, more preferably 3 to 15 wt.% and still more preferably 5 to 12 wt.% of the total weight of the oral dosage form.

Surfactants usually are substances, which lower the interfacial tension between two phases, thus enabling or supporting the formation of dispersions or working as a solubilizer. Common surfactants can be alkyl sulfates (for example sodium lauryl sulfate), alkyltrimethylammonium salts, alcohol ethoxylates, sorbitanes and the like. Sorbitans are preferred and sorbitan monododecanoate is especially preferred.

The surfactant can be present in the oral dosage form of the present invention in an amount of 0 to 10 wt.%, preferably 0.1 to 8 wt.%, more preferably 0.3 to 5 wt.% and still more preferably 0.7 to 4.0 wt.% of the total weight of the oral dosage form.

Lubricants are generally used in order to reduce sliding friction. In particular, the intention is to reduce the sliding friction found during tablet pressing between the punch moving up and down in the die and the die wall on the one hand, and between the edge of the tablet and the die wall on the other hand. Suitable lubricants are, for example, stearic acid, adipic acid, sodium stearyl fumarate and/or magnesium stearate. Sodium stearyl fumarate is particularly preferred.

Lubricants are generally used in an amount of up to 3% by weight, preferably 0.1 to 2 wt.%, based on the total weight of the dosage form.

It lies in the nature of pharmaceutical excipients that they sometimes can perform more than one function in a pharmaceutical formulation. Therefore, the vehicle (c) may act as excipient (d) and vice versa. For example, povidone may act both as vehicle and binder. However, in order to provide an unambiguous delimitation, the fiction will therefore preferably apply that a substance, which is used as a particular excipient, is not simultaneously also used as a further pharmaceutical excipient. For example, microcrystalline cellulose - if used as a vehicle (c) - is not also used for example as a disintegrant (even though microcrystalline cellulose also exhibits a certain disintegrating effect).

In another embodiment of the invention, the mixture of the non-crystalline lopinavir (a) and the vehicle (c) is obtained by a melt process. For this purpose, lopinavir, preferably crystalline lopinavir, is melted in the present of the vehicle (c), wherein it is assured that the lopinavir is obtained in a non-crystalline form. Alternatively, the mixture of the non-crystalline lopinavir (a) and the vehicle (c) can be obtained by a melt process. With regard to preferred embodiments of these features of the dosage form of the present invention, it is referred to the explanations given below for the process of the present invention.

In a preferred embodiment the oral dosage form of the invention can preferably comprise an intragranular phase comprising non-crystalline lopinavir (a), optionally vehicle (c) and one or more excipient(s) (d), and an extragranular phase comprising crystalline ritonavir (b), optionally vehicle (c) and further excipient(s) (d).

In an alternative preferred embodiment the extragranular phase can further comprise one or more active agent(s), preferably selected from zidovudine, lamivudine, tenofovir and/or abacavir.

In case the intragranular phase comprises non-crystalline lopinavir (a) and an inorganic substance as vehicle (c), the intragranular phase preferably does not comprise any further excipients. In particular, in this case the intragranular phase preferably does not comprise a water-soluble polymer.

The extragranular phase can preferably comprise more vehicle(s) (c). In a preferred embodiment, the extragranular phase preferably comprises at least one non-brittle vehicle and at least one brittle vehicle. The at least one non-brittle vehicle (c), comprised in the extragranular phase, can preferably be an organic polymer which preferably can also have binding properties. For example, the non-brittle vehicle (c) in the extragranular phase can preferably be polyvinylyrrolidone, HPMC or a vinylpyrrolidone vinyl acetate copolymer, e.g. with a weight average molecular weight of 25,000 to 80,000 g/mol. Vinylpyrrolidone vinylacetate copolymer is particularly preferred.

In a preferred embodiment the oral dosage form of the present invention can preferably comprise the following amounts of components:
5 to 40 wt.%, preferably 10 to 35 wt.%, more preferably 15 to 25 wt.% lopinavir (a),
1 to 10 wt.%, preferably 2 to 9 wt.%, more preferably 4 to 8 wt.% ritonavir (b),
5 to 75 wt.%, preferably 10 to 60 wt.%, more preferably 15 to 45 wt.% vehicle (c),
0 to 1 wt.%, preferably 0.01 to 0.8 wt.%, more preferably 0.02 to 0.5 wt.% glidant
0 to 40 wt.%, preferably 10 to 35 wt.%, more preferably 15 to 30 wt.% filler,
0 to 20 wt.%, preferably 3 to 17 wt.%, more preferably 5 to 12 wt.% disintegrant,
0 to 20 wt.%, preferably 2 to 15 wt.%, more preferably 3 to 10 wt.% surfactant,
0 to 3 wt.%, preferably 0.3 to 2.5 wt.%, more preferably 0.5 to 2.0 wt.% lubricant,
wherein the wt.% are based on the total weight of the dosage form.

In a preferred embodiment, the oral dosage form of the present invention preferably comprises:
an internal phase comprising
   5 to 40 wt.%, preferably 10 to 35 wt.%, more preferably 15 to 25 wt.% lopinavir (a),
   2 to 55 wt.%, preferably 5 to 45 wt.%, more preferably 10 to 35 wt.% vehicle (c), wherein the vehicle (c) preferably is a brittle substance and/or an inorganic substance, more preferably an aluminosilicate, and further preferably does not comprise a water-soluble polymer;
and an external phase comprising
   1 to 10 wt.%, preferably 2 to 9 wt.%, more preferably 4 to 8 wt.% ritonavir (b),
   0 to 50 wt.%, preferably 2 to 45 wt.%, more preferably 5 to 40 wt.% vehicle (c), wherein the vehicle (c) preferably comprises a non-brittle substance, more preferably an hydrophilic polymer, in particularly povidone or copovidone or HPMC, and, optionally, a brittle substance, preferably an aluminosilicate or microcrystalline cellulose, more preferably microcrystalline cellulose, wherein the ratio of non-brittle substance to brittle substance is preferably between 4 : 10 to 1 : 25,
   0 to 1 wt.%, preferably 0.01 to 0.8 wt.%, more preferably 0.02 to 0.5 wt.% glidant,
   0 to 40 wt.%, preferably 10 to 35 wt.%, more preferably 15 to 30 wt.% filler,
   0 to 20 wt.%, preferably 3 to 17 wt.%, more preferably 5 to 12 wt.% disintegrant,
   0 to 20 wt.%, preferably 2 to 15 wt.%, more preferably 3 to 10 wt.% surfactant, in particular, sorbitane monododecanoate,
   0 to 3 wt.%, preferably 0.3 to 2.5 wt.%, more preferably 0.5 to 2.0 wt.% lubricant,
   wherein the wt.% are based on the total weight of the dosage form.

In a preferred embodiment the oral dosage form of the present invention is in the form of a capsule or a tablet. In case of the form of a capsule, the present dosage form is preferably in the form of a hard-shell or soft-shell capsule. Alternatively, the dosage form can be present in form of a powder or preferably granulate, which is stored in a sachet or stick-pack.

In particular, the oral dosage form of the present invention is a tablet, preferably a tablet for peroral use. Alternatively, it could be a dispersing tablet or an oral dispersible tablet (ODT).

Another subject of the present invention is a method for preparing an oral dosage form according to the present invention comprising the steps of
(i) providing lopinavir, optionally vehicle (c) and/or pharmaceutical excipient (d),
(ii) optionally processing the mixture of step (i) to assure that lopinavir is obtained in a non-crystalline form,
(iii) optionally granulating the lopinavir of step (i) or the mixture of step (ii),
(iv) mixing the mixture of step (i) or the processed mixture of step (ii) or the granules of step (iii) with crystalline ritonavir (b) and further vehicles (c) and/or one or more pharmaceutical excipients (d),
(v) processing the mixture of step (iv) into an oral dosage form.

Generally, in step (i) lopinavir, preferably crystalline lopinavir, can be present in an amount of 20 to 100 wt.%, preferably 25 to 60 wt.%, more preferably 30 to 55 wt.%, and particularly preferred between 33 and 51 wt.%, based on the total weight of the mixture resulting from step (i).

Generally, in step (i), vehicle (c) can be present in an amount of 0 to 80 wt.%, preferably 40 to 75 wt.%, more preferably 45 to 70 wt.%, and particularly preferred between 49 and 67 wt.%, based on the total weight of the mixture resulting from step (i).

Especially preferred vehicles (c) in these embodiments can be brittle and/or inorganic substances, preferably silicates, more preferably magnesium aluminosilicates. If so, in step (i) the amount of excipient (d) can be preferably 0 wt.%.

In an alternative embodiment the vehicle (c) can preferably be an organic polymer. If so, excipient (d) can be preferably a glidant. The glidant, such a fumed silica, can be preferably present in an amount of 0 to 20 wt.%, preferably 0.5 to 15 wt.%, more preferably 1 to 12 wt.%, and particularly preferred between 1.5 and 10 wt.%, based on the total weight of the mixture resulting from step (i).

In a preferred embodiment, the provision in step (i) can be carried out with conventional mixing devices, e.g. in a free fall mixer like Turbula^{®} T 10B (Bachofen AG, Switzerland). Mixing can be carried out, e.g., for 1 minute to 1 hour, preferably for 5 to 30 minutes.

In an alternative embodiment, in step (I) lopinavir can preferably be provided in non-crystalline form. In that case, the provision of a vehicle and/or pharmaceutical excipient can preferably be omitted.

In optional step (ii) the mixture of step (i) is processed in order to assure that lopinavir is obtained in a non-crystalline form. This means, if in step (i) lopinavir is employed in non-crystalline form, then step (ii) might be omitted. However, if in step (i) lopinavir is employed in crystalline form, then step (ii) is necessary. In the later case, the process conditions of step (ii) have to be chosen such that crystalline lopinavir is transformed into non-crystalline lopinavir. In a preferred embodiment process step (ii) preferably comprise a melt process.

In a preferred embodiment the optional process step (ii) can be a melt process, especially a melt extrusion or a melt granulation process.

In a preferred embodiment of the melting step (ii) the vehicle (c) can be an inorganic substance, preferably a silicate, more preferably a magnesium aluminosilicate. The melting conditions are preferably selected such that the lopinavir is obtained in a non-crystalline form. The specific melting conditions can preferably depend on the melting point of lopinavir. Therefore, temperatures for example should be from 1°C to 10°C, preferably from 2°C to 5°C, higher than the melting point of lopinavir. In a preferred embodiment, during the melting step (ii) temperatures from 80 to 160°C, preferably from 100 to 150°C, more preferably from 120 to 140°C, are used.

In an alternative embodiment of the melting process, the vehicle (c) can be preferably an organic polymer. Again, the melting conditions are preferably selected such that the lopinavir is obtained in a non-crystalline form. The specific melting conditions can depend on amount and kind of vehicle (c). Usually, for the melting step temperatures are applied, which are between the glass transition point Tg of the vehicle and an upper temperature limit of about to 200°C. Preferably, the temperature is between Tg of the vehicle +5°C and 150°C, more preferably between Tg of the vehicle + 10°C and 130°C.

Preferably, lopinavir and the vehicle (c) are chosen in such amounts that an eutectic mixture results.

It was unexpectedly found that melting only one of the active agents, namely lopinavir, instead of both active agents significantly increases the desirable properties of the resulting dosage form.
Mixing of the substances provided in step (i) preferably should be conducted before the melting process.

After the melting process the molten mixture can be cooled off. In a preferred embodiment, the cooling off step of the molten lopinavir or lopinavir/vehicle mixture is conducted under cooling conditions, chosen such that non-crystalline lopinavir remains in a non-crystalline form. Preferably, the cooling rate is 5 to 50°C per minute, in particular 45 to 50°C per minute.

In a preferred embodiment, in optional step (iii) the mixture resulting from melting step (ii) is granulated. In case of a melting step (ii) the mixture can be granulated either in the molten state or preferably after having cooled off.
Step (iii) of granulating the mixture, which results from melting step (II), can be preferably carried out, for example, by an extrusion process. Hence, steps (ii) and (iii) preferably can be regarded as melt-extrusion or melt-granulation processes. Generally, the extrusion process should be capable of providing essentially spherical particles. Suitable extruders are, for example, screw-feed extruders (axial or endplate, dome and radial) or gravity extruders (cylinder roll, gear roll or radial). Screw-feed extruders are preferred.

The granulation can also, for example, be carried out by a - preferably heatable - High-Shear-Mixer (e.g. Diosna^{®} P1/6). In this case, steps (i), (ii) and (iii) can be regarded as one process with different sequences of special parameters. The first sequence can be step (i) without heating, the second sequence can be a mixture of step (i) and step (ii) with heating, sequence three can include parts of step (ii) and step (iii). Preferred parameters of the sequences can be dependent on the lopinavir and the vehicle (c), preferably a silicate, more preferably a magnesium aluminosilicate.

In a preferred embodiment the granulation can be carried out with a melt screw extruder (e.g. Thermo Fisher^{®} Eurolab 16), wherein steps (i) and (iii) can be unified in one continuous process. Generally, a temperature gradient can be applied, preferably between 45°C to 180°C, more preferably between 55°C to 150°C, in particular between 65°C to 130°C.

In a preferred embodiment the granulation conditions in step (iii) are chosen such that the resulting granulated pharmaceutical composition can comprise an average particle size (D50) of 10 to 500 µm, more preferably of 30 to 250 µm, furthermore preferably of 50 to 200 µm, most preferably of 70 to 170 µm.

The bulk density of the granulated pharmaceutical composition resulting from step (iii) of the process of the present invention can usually range from 0.2 to 0.85 g/ml, preferably from 0.25 to 0.85 g/ml, more preferably from 0.3 to 0.75 g/ml.

The mixture of step (ii) or the granules of step (iii), comprising non-crystalline lopinavir, can be regarded as "intragranular phase".

In step (iv), the mixture from step (i) (when lopinavir was provided in non-crystalline form), the processed mixture of step (ii) or the granules of step (iii) are mixed with crystalline ritonavir and optionally vehicle (c) and/or further excipient(s) (d).

The mixing (iv) can be carried out with mixing devices, e.g. in a free fall mixer like Turbula^{®} T 10B (Bachofen AG, Switzerland). Mixing can be carried out for example for 1 minute to 1 hour, preferably for 5 to 30 minutes.

In a preferred embodiment the vehicle (c) used in the mixing step (iv) can preferably be the same vehicle (c) or mixture of vehicle as used for the preparation of the mixture containing non-crystalline lopinavir (a) and vehicle (c).

With regard to the excipient(s) (d) used in the step (iv), it is referred to the above mentioned pharmaceutically acceptable excipient(s) (d).

In step (v), the mixture of step (iv) is processed into an oral dosage form. Step (v) can comprise, for example, compressing the mixture of step (iv) into tablets or filling mixture of step (iv) into capsules, sachets or stick-packs. Preferably the mixture is compressed into tablets.

In an embodiment, the processing of the mixture of step (iv) into an oral dosage form can be done by filling the mixture of step (iv) into capsules, preferably hard shell capsules. For this filling of the mixture of step (iv) into capsules, dependent dosing systems (for example an auger) or preferably independent dosing systems (for example MG2, Matic (IMA)) can be used.

In a preferred embodiment, the mixture of step (iv) is compressed into tablets, for example, on a rotary press, e.g. on a Fette^{®} (Fette GmbH, Germany) or a Riva^{®} Piccola (Riva, Argentina) or an eccentric press, e.g. a Korsch^{®} EKO. The compression force usually ranges from 1 to 50 kN, preferably 3 to 40 kN. The resulting tablets preferably have a hardness of 30 to 400 N, more preferred 50 to 325 N, still more preferred from 65 to 275 N, in particular from 85 to 225 N, wherein the hardness is measured according to Ph. Eur., 6.0, Chapter 2.9.8.

Further, the tablets of the invention preferably have contents of active agent(s), which lie within the concentration of 90 to 110%, preferably 95 to 105%, especially preferred from 98 to 102% of the average content of the active agents(s). This "content uniformity" is determined with a test in accordance with Ph. Eur., 6.0, Chapter 2.9.6. According to that test, the content of the active agents of each individual tablet out of 20 tablets must lie between of 90 to 110%, preferably 95 to 105%, especially 98 to 102% of the average content of the active agents(s). Therefore, the content of the active drugs in each tablet of the invention differs from the average content of the active agent by at most 10%, preferably at most 5 and especially at most 2%.

In addition, the resulting tablets preferably have a friability of less than 5%, particularly preferably less than 2%, especially less than 1%. The friability is determined in accordance with Ph. Eur., 6.0, Chapter 2.9.7. The friability of tablets generally refers to tablets without coating.

The dosage form of the invention tablets may be a peroral tablet, which can be swallowed unchewed. The tablet can preferably be film-coated.

Generally, film-coatings, which do not affect the release of the active agent(s) and film-coatings affecting the release of the active agent(s), can be employed with tablets according to invention. The film-coatings, which do not affect the release of the active agent(s), are preferred.

Preferred examples of film-coatings, which do not affect the release of the active ingredient, can be those including poly(meth)acrylate, methylcellulose (MC), hydroxypropyl methylcellulose (HPMC), hydroxypropyl cellulose (HPC), hydroxyethyl cellulose (HEC), polyvinyl pyrrolidone (PVP) and mixtures thereof. These polymers can have a weight-average molecular weight of 10,000 to 150,000 g/mol.

In an alternative preferred embodiment, the film-coating can affect the release of the active agent. Examples for film-coating, affecting the release of the active agent, are gastric juice resistant film-coatings and retard coatings.

Further the coating can be free from active ingredient. However, it is also possible that the coating can contain an active ingredient (lopinavir and/or ritonavir, preferably only ritonavir). In such a case, this amount of active ingredient would function as an initial dose. In such a case, the coating preferably can comprise 1 to 45 wt.%, preferably 5 to 35 wt.%, most preferably 10 to 30 wt.% of lopinavir or ritonavir, based on the total amount of lopinavir or ritonavir contained in the tablet.

In the preferred case that the film coating does not contain an active agent (a) or (b), said coating can have a thickness of 2 µm to 100 µm, preferably from 20 to 60 µm. In case of a coating containing an active agent (a) or (b), the thickness of the coating is usually 10 µm to 200 µm, preferably from 50 to 125 µm.

The oral dosage form of the present invention can preferably be employed in the treatment and prevention of infection caused by viruses, especially infection caused by HIV viruses.

When treating the diseases which are indicated for the active agent or the combination of active agents in the oral dosage forms of the invention, satisfactory results are usually obtained when lopinavir contained in the dosage form is administered in a daily dose of 100 to 1000 mg, preferably 160 to 960 mg, more preferably 200 to 900 mg and particularly 400 to 800 mg. For the same purpose, ritonavir contained in the dosage form is administered in a daily dose of 25 to 250 mg, preferably 40 to 240 mg, more preferably 50 to 225 mg and particularly 100 to 200 mg. In the same doses, applications less than once a day are possible, such as every two, three or four days, for example in a delayed-release formulation. The dosing regimen may be varied within or even outside this frame in order to achieve the optimum treatment results.

In a preferred embodiment the composition and/ or the dosage form according to the invention provides an immediate release ("IR") of lopinavir/ritonavir. This means that the release profile of the dosage form of the invention according to USP method (paddle, 900 ml, water with 0.06 M C12E10 (polyoxyethylene-10-lauryl ether), 75 rpm, 37°C) after 10 minutes usually indicates a content release of at least 20%, after 20 minutes a content release of at least 30%, after 30 minutes a content release of at least 45% and after 45 minutes a content release of at least 55%.

### EXAMPLES

### Hot Melt Extrusion

### Example 1

Crystalline lopinavir was mixed with magnesium aluminosilicate, Al₂O₃·MgO·1.7SiO₂·xH₂O. The powdery mixture was then fed into a Thermo Scientific *Pharma 16 win-screw extruder at a melt temperature of 130°C. During the melting step, a complete conversion into amorphous lopinavir occurred. The extrudate was cut into pieces and allowed to solidify. The extruded pieces were milled using a high impact universal mill Quadro Comil Underdriven^{®} with a 800 µm rasp sieve. The milled material was blended in a Turbula^{®} T10B Shaker-Mixer with ritonavir, microcrystalline cellulose (Avicel^{®} PH 102), copovidone (Kollidon^{®} VA 64) and crospovidone (Kollidon^{®} CL) for 15 minutes. After addition of sodium stearyl fumarate and blending for further 5 minutes, the powdery blend was compressed on an eccentric press Korsch^{®} EKO to 21 mm oblong tablets (825 mg) with a hardness of approximately 150 N each, containing

**Intraganular**

| | |
|---|---|
| Lopinavir | 200 mg |
| Al₂O₃·MgO·1.7SiO₂·xH₂O | 200 mg |
| | |
| Extragranular | |
| Ritonavir | 50 mg |
| Microcrystalline cellulose | 240 mg |
| Copovidone | 60 mg |
| Crospovidone | 60 mg |
| Sodium stearyl fumarate | 15 mg |

**Parameters of hot melt extrusion**

| zone | die | zone 10 | zone 9 | zone 8 | zone 7 | zone 6 | zone 5 | zone 4 | zone 3 | zone 2 | Dosing |
|---|---|---|---|---|---|---|---|---|---|---|---|
| target temp. [°C] | 20 | 130 | 130 | 130 | 130 | 130 | 130 | 130 | 130 | 70 | 20 |

| screw speed [rpm] | solid dosing [%] | torque [%] | mass pressure [bar] |
|---|---|---|---|
| 30 | 5.00 | 30.00 | --- |

### Example 2

Crystalline lopinavir was mixed with magnesium aluminosilicate, Al₂O₃·MgO·1.7SiO₂·xH₂O. The powdery mixture was then fed into a Thermo Scientific *Pharma 16 win-screw extruder at a melt temperature of 130°C. During the melting step, a complete conversion into amorphous lopinavir occurred. The extrudate was cut into pieces and allowed to solidify. The extruded pieces were milled, using a high impact universal mill Quadro Comil Underdriven^{®} with a 800 µm rasp sieve. All ingredients, except of sodium stearyl fumarate, were blended in a Turbula^{®} T10B Shaker-Mixer for 15 minutes. Sorbitan laurate (Span^{®} 20) was incorporated prior by granulation with microcrystalline cellulose (Avicel^{®} PH 102) and lactose monohydrate + Povidone (Ludipress^{®} LCE) in a Diosna^{®} P1-6-high-sheer mixer. After addition of sodium stearyl fumarate and blending for further 5 minutes, the powdery blend was compressed on an eccentric press Korsch^{®} EKO to 21 mm oblong tablets (1025 mg) with a hardness of approximately 150 N each, containing

**Intraganular**

| | |
|---|---|
| Lopinavir | 200 mg |
| Al₂O₃·MgO·1.7SiO₂·xH₂O | 200 mg |

**Extragranular**

| | |
|---|---|
| Ritonavir | 50 mg |
| Microcrystalline cellulose | 240 mg |
| Lactose monohydrate +Povidone | 160 mg |
| Sorbitan laurate | 40 mg |
| Copovidone | 60 mg |
| Crospovidone | 60 mg |
| Sodium stearyl fumarate | 15 mg |

**Parameters of hot melt extrusion**

| zone | die | zone 10 | zone 9 | zone 8 | zone 7 | zone 6 | zone 5 | zone 4 | zone 3 | zone 2 | Dosing |
|---|---|---|---|---|---|---|---|---|---|---|---|
| target temp. [°C] | 20 | 130 | 130 | 130 | 130 | 130 | 130 | 130 | 130 | 70 | 20 |

| screw speed [rpm] | solid dosing [%] | torque [%] | mass pressure [bar] |
|---|---|---|---|
| 30 | 5.00 | 30.00 | --- |

### Comparative Example

The comparative example corresponds to Example 3 of patent application EP 1 663 183 B1.

Copovidone was blended with sorbitan monolaurate (Span 20) in a Diosna high-shear mixer. The resulting granules were mixed with ritonavir, lopinavir and colloidal silica. The powdery mixture was then fed into a twin-screw extruder with a melt temperature of 119°C. The extrudate was cut into pieces and allowed to solidify. The extruded pieces were milled using a co-mill from Retsch. The milled material was blended with sodium stearyl fumarate and colloidal silica for 10 minutes. The powdery blend was compressed on an eccentric press EK0 from Korsch to tablets (601 mg), each containing

| | |
|---|---|
| Lopinavir | 100 mg |
| Ritonavir | 25 mg |
| Copovidone | 427 mg |
| Sorbitan monolaurate | 42 mg |
| Collodial silica | 6 mg |
| Sodium stearyl fumarate | 1 mg |

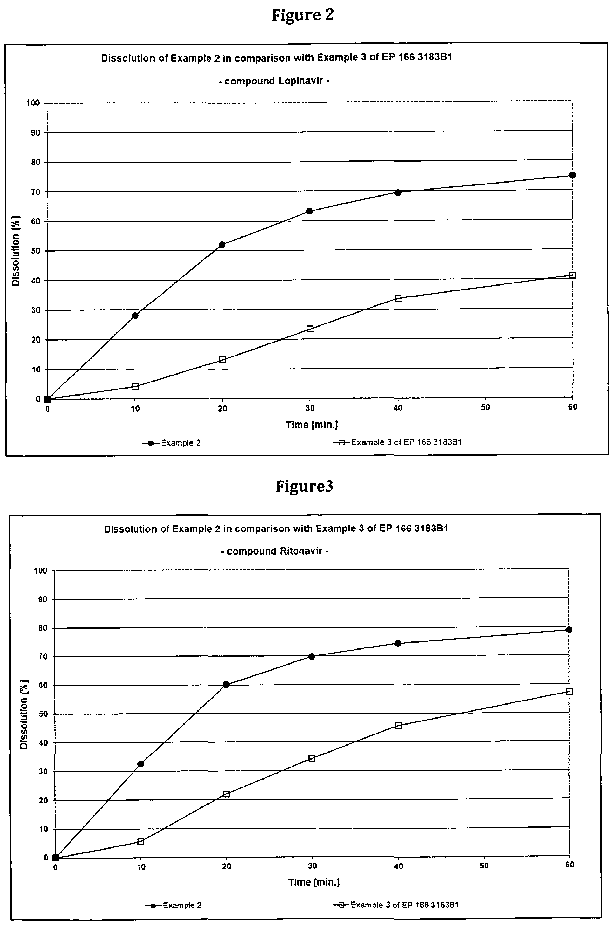

As can be seen from Figures 2 and 3, the tablet according to present Example 2 shows superior dissolution profiles for both lopinavir and ritonavir compared to the tablet prepared according to example 3 of EP 1 663 183 B1. In particular,the dissolution profiles of both active agents of the present tablet do not show any lag time.

## Claims

1. Oral dosage form comprising
(a non-crystalline lopinavir, and
(b) crystalline ritonavir.

2. Oral dosage form according to claim 1, wherein the non-crystalline lopinavir (a) is present in a mixture with a vehicle (c).

3. Oral dosage form according to claim 2, wherein the weight ratio of non-crystalline lopinavir (a) to vehicle (c) is from 1: 5 to 5 : 1.

4. Oral dosage form according to claim 2 or 3, wherein the vehicle (c) is an organic polymer or an inorganic substance.

5. Oral dosage form according to any one of claims 2 to 4, wherein the vehicle (c) is a silicate.

6. Oral dosage form according to claim 5, wherein the vehicle (c) possesses a specific surface area from 75 to 350 m²/g, the specific surface area being measured according to Ph.Eur. 6.0, 2.9.26.

7. Oral dosage form according to any one of claims 2 to 4, wherein the vehicle (c) is a brittle substance, having a yield pressure from 80 MPa to 500 MPa.

8. Oral dosage form according to any one of claims 2 to 7, wherein the mixture of the non-crystalline lopinavir (a) and the vehicle (c) are obtained by an extrusion process.

9. Oral dosage form according to any one of claims 1 to 8, wherein the dosage form comprises
an intragranular phase comprising non-crystalline lopinavir (a), optionally vehicle (c) and/or excipient (d); and
an extragranular phase comprising crystalline ritonavir (b), optionally vehicle (c) and/or further excipient(s) (d).

10. Oral dosage form according to claim 9, wherein the intragranular phase comprises a brittle vehicle (c), and the extragranular phase comprises a non-brittle vehicle (c) and a surfactant.

11. Oral dosage form according to any one of claims 1 to 10, wherein the dosage form comprises
10 to 35 wt.% lopinavir (a),
2 to 9 wt.% ritonavir (b),
10 to 50 wt.% vehicle (c),
0 to 0.8 wt.% glidant,
10 to 35 wt.% filler,
3 to 17 wt.% disintegrant,
2 to 15 wt.% surfactant,
0.3 to 2.5 wt.% lubricant,
based on the total weight of the dosage form.

12. Oral dosage form according to any one of claims 1 to 11, wherein the oral dosage form is in form of a capsule or a tablet.

13. A method for preparing an oral dosage form according to any one of claims 1 to 12 comprising
(i) providing lopinavir, optionally vehicle (c) and/or pharmaceutical excipient(s) (d),
(ii) optionally processing the mixture of step (i) to assure that lopinavir is obtained in a non-crystalline form,
(iii) optionally granulating the pharmaceutical composition from step (ii),
(iv) mixing the lopinavir of step (i), the mixture of step (ii) or the granules of step (iii) with crystalline ritonavir and optionally vehicle (c) and/or pharmaceutical excipient(s) (d),
(v) processing the mixture of step (iv) into an oral dosage form.

14. A method according to claim 13, wherein processing of step (ii) comprises a melting process.

## Patentansprüche

1. Orale Darreichungsform umfassend
(a) nichtkristallines Lopinavir und
(b) kristallines Ritonavir.

2. Orale Darreichungsform gemäß Anspruch 1, wobei das nichtkristalline Lopinavir (a) in einem Gemisch mit einem Schlepper (c) vorliegt.

3. Orale Darreichungsform gemäß Anspruch 2, wobei das Gewichtsverhältnis von nichtkristallinem Lopinavir (a) zu Schlepper (c) 1 : 5 bis 5 : 1 beträgt.

4. Orale Darreichungsform gemäß Anspruch 2 oder 3, wobei es sich bei dem Schlepper (c) um ein organisches Polymer oder einen anorganischen Stoff handelt.

5. Orale Darreichungsform gemäß einem der Ansprüche 2 bis 4, wobei es sich bei dem Schlepper (c) um ein Silicat handelt.

6. Orale Darreichungsform gemäß Anspruch 5, wobei der Schlepper (c) eine spezifische Oberfläche von 75 bis 350 m²/g aufweist, wobei die spezifische Oberfläche gemäß Ph.Eur. 6.0, 2.9.26 gemessen ist.

7. Orale Darreichungsform gemäß einem der Ansprüche 2 bis 4, wobei es sich bei dem Schlepper (c) um einen spröden Stoff handelt, der einen Fließdruck von 80 MPa bis 500 MPa aufweist.

8. Orale Darreichungsform gemäß einem der Ansprüche 2 bis 7, wobei das Gemisch von nichtkristallinem Lopinavir (a) und dem Schlepper (c) durch ein Extrusionsverfahren erhalten wird.

9. Orale Darreichungsform gemäß einem der Ansprüche 1 bis 8, wobei die Darreichungsform umfasst:
eine intragranuläre Phase, umfassend nichtkristallines Lopinavir (a),
gegebenenfalls einen Schlepper (c) und/oder einen Hilfsstoff (d); und
eine extragranuläre Phase, umfassend kristallines Ritonavir (b),
gegebenenfalls einen Schlepper (c) und/oder weitere Hilfsstoff(e) (d).

10. Orale Darreichungsform gemäß Anspruch 9, wobei die intragranuläre Phase einen spröden Schlepper (c) umfasst und die extragranuläre Phase einen nichtspröden Schlepper (c) und ein grenzflächenaktives Mittel umfasst.

11. Orale Darreichungsform gemäß einem der Ansprüche 1 bis 10, wobei die Darreichungsform umfasst:
10 bis 35 Gew.-% Lopinavir (a),
2 bis 9 Gew.-% Ritonavir (b),
10 bis 50 Gew.-% Schlepper (c),
0.01 bis 0.8 Gew.-% Fließregulierungsmittel,
10 bis 35 Gew-.% Füllstoff,
3 bis 17 Gew.-% Sprengmittel,
2 bis 15 Gew.-% grenzflächenaktives Mittel,
0.3 bis 2.5 Gew.-% Schmiermittel,
bezogen auf das Gesamtgewicht der Darreichungsform.

12. Orale Darreichungsform gemäß einem der Ansprüche 1 bis 11, wobei die orale Darreichungsform in der Form einer Kapsel oder einer Tablette vorliegt.

13. Verfahren zum Herstellen einer oralen Darreichungsform gemäß einem der Ansprüche 1 bis 12, umfassend
(i) Bereitstellen von Lopinavir, gegebenenfalls Schlepper (c) und/oder pharmazeutischem Hilfsstoff oder Hilfsstoffen (d),
(ii) gegebenenfalls Verarbeiten des Gemischs aus Schritt (i), um sicherzustellen, dass Lopinavir in einer nichtkristallinen Form erhalten wird,
(iii) gegebenenfalls Granulieren der pharmazeutischen Zusammensetzung aus Schritt (ii),
(iv) Mischen des Lopinavirs aus Schritt (i), des Gemischs aus Schritt (ii) oder der Granulatkörner aus Schritt (iii) mit kristallinem Ritonavir und gegebenenfalls einem Schlepper (c) und/oder einem pharmazeutischen Hilfsstoff oder Hilfsstoffen (d),
(v) Verarbeiten des Gemischs (iv) zu einer oralen Darreichungsform.

14. Verfahren gemäß Anspruch 13, wobei das Verarbeiten bei Schritt (ii) ein Schmelzverfahren umfasst.

## Revendications

1. Forme posologique orale comprenant
(a) du lopinavir non-cristallin, et
(b) du ritonavir cristallin.

2. Forme posologique orale selon la revendication 1, dans laquelle le lopinavir non-cristallin (a) est présent en un mélange avec un véhicule (c).

3. Forme posologique orale selon la revendication 2, dans laquelle le rapport de poids du lopinavir non-cristallin (a) au véhicule (c) est de 1 : 5 à 5 : 1.

4. Forme posologique orale selon la revendication 2 ou 3, dans laquelle le véhicule (c) est un polymère organique ou une substance inorganique.

5. Forme posologique orale selon l'une quelconque des revendications 2 à 4, dans laquelle le véhicule (c) est un silicate.

6. Forme posologique orale selon la revendication 5, dans laquelle le véhicule (c) possède une superficie spécifique de 75 à 350 m²/g, la superficie spécifique étant mesurée selon Ph.Eur. 6.0 , 2.9.26.

7. Forme posologique orale selon l'une quelconque des revendications 2 à 4, dans laquelle le véhicule (c) est une substance cassante, ayant une pression de rupture de 80 MPa à 500 MPa.

8. Forme posologique orale selon l'une quelconque des revendications 2 à 7, dans laquelle le mélange du lopinavir non-cristallin (a) et du véhicule (c) est obtenu par un procédé d'extrusion.

9. Forme posologique orale selon l'une quelconque des revendications 1 à 8, dans laquelle la forme posologique comprend
une phase intragranulaire comprenant du lopinavir non-cristallin (a), facultativement du véhicule (c) et/ou de l'excipient (d); et
une phase extragranulaire comprenant du ritonavir cristallin (b), facultativement du véhicule (c) et/ou un ou plusieurs autre(s) excipient(s) (d).

10. Forme posologique orale selon la revendication 9, dans laquelle la phase intragranulaire comprend un véhicule cassant (c), et la phase extragranulaire comprend un véhicule non-cassant (c) et un tensioactif.

11. Forme posologique orale selon l'une quelconque des revendications 1 à 10, dans laquelle la forme posologique comprend
10 à 35 % en poids de lopinavir (a),
2 à 9 % en poids de ritonavir (b),
10 à 50 % en poids de véhicule (c),
0 à 0,8 % en poids d'agent de glissement,
10 à 35 % en poids de substance de remplissage,
3 à 17 % en poids de désintégrant,
2 à 15 % en poids de tensioactif,
0,3 à 2,5 % en poids de lubrifiant,
sur la base du poids total de la forme posologique.

12. Forme posologique orale selon l'une quelconque des revendications 1 à 11, dans laquelle la forme posologique orale est en forme d'une capsule ou d'un comprimé.

13. Un procédé pour produire une forme posologique orale selon l'une quelconque des revendications 1 à 12 comprenant
(i) la provision de lopinavir, facultativement de véhicule (c) et/ou d'excipient(s) pharmaceutique(s) (d),
(ii) facultativement le traitement du mélange de l'étape (i) afin d'assurer que le lopinavir est obtenu en une forme non-cristalline,
(iii) facultativement la granulation de la composition pharmaceutique de l'étape (ii),
(iv) le mélange du lopinavir de l'étape (i), du mélange de l'étape (ii) ou des granules de l'étape (iii) avec du ritonavir cristallin et facultativement du véhicule (c) et/ou un ou plusieurs excipient(s) pharmaceutique(s) (d),
(v) la transformation du mélange de l'étape (iv) en une forme posologique orale.

14. Un procédé selon la revendication 13, dans lequel le traitement de l'étape (ii) comprend un processus de fusion.
